# EUROPEAN PATENT APPLICATION

(11) **EP 2 392 924 A1**
(43) Date of publication of application: **07.12.2011**
(21) Application number: 11173497.6
(22) Date of filing: 03.11.2006
(51) Int. Cl.: G01N 33/536, G01N 33/68, G01N 33/543

(54) **Rapid ELISA**

(30) Priority: 04.11.2005 GB 0522600; 06.02.2006 GB 0602336
(62) Divisional of application: 06847258.8
(71) Applicant: Novartis Vaccines and Diagnostics S.r.l., 53100 Siena (SI) (IT)
(72) Inventor: Berti, Duccio, I -53100 Siena (IT); Casini, Daniele, I-53100 Siena (IT); Fontani, Paola, I-53100 Siena (IT)
(74) Representative: Marshall, Cameron John

(57) **Abstract**

A step in the enzyme-linked immunosorbent assay (ELISA) is changed from a heterogeneous phase to a homogeneous phase, to give a much shorter overall completion time. The new assay comprises the steps of (i) mixing in a homogeneous phase a sample and a first binding partner to an analyte to form a first mixing product; and (ii) exposing the first mixing product to a second binding partner to the analyte.

## Description

### TECHNICAL FIELD

This invention is in the field of enzyme linked immunosorbent assays (ELISAs).

### BACKGROUND ART

ELISA is one of the most commonly used methods for the detection of and quantitation of antibodies, antigens, hormones, cytokines, synthetic peptides and other molecules (Engvall E. 1980 Methods Enzymol. 70:419-39).

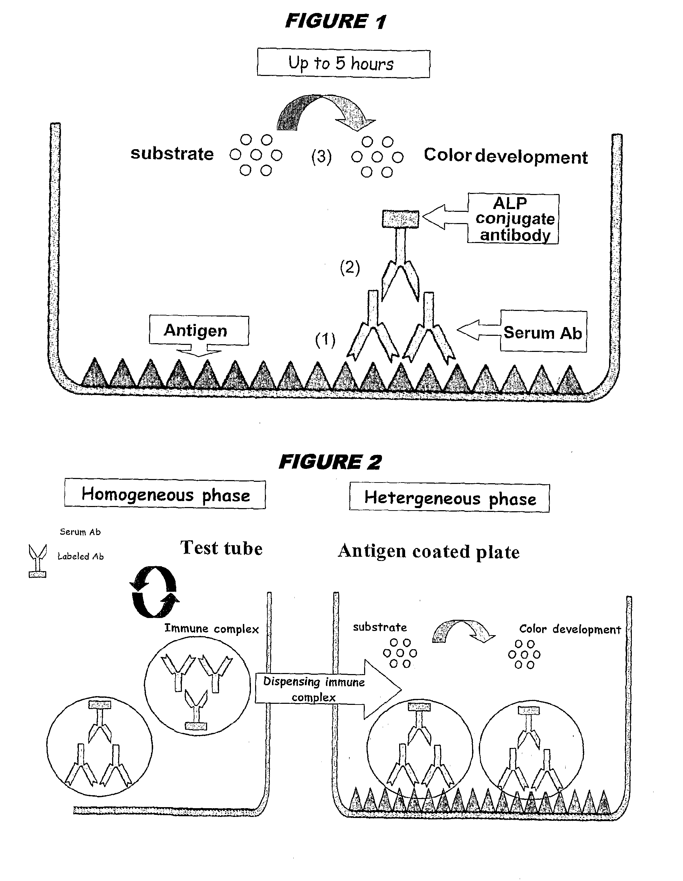

Very often ELISA is used as the exclusive method not only for detecting and quantitation of a variety of ligands but also for studying their molecular interactions. Indeed, the assay is extremely popular both in research and in the clinic due to the ability to test a large number of samples, and because it does not involve radioactivity. In carrying out the procedure, a ligand (usually in the form of an antigen, antibody or peptide) is coated onto the surface of each micro-well in a 96-well micro plate and held in place via non-covalent bonds between the hydrophobic regions of the protein and the non-polar plastic surface. The residual, non-coated area of each well is then blocked with an inert protein or polymer to prevent any non-specific binding of the reagents. Next, the corresponding counter molecule is incubated with the ligand over a period of several hours. The well is then washed and an enzyme conjugated second antibody, specific to the counter molecule, is added and incubated for approximately 2hrs or more. Finally, a substrate for the conjugated enzyme is provided and the reaction is quantified by measuring any colour change in the substrate. Figure 1 shows the steps in a standard ELISA test for assaying serum antibodies.

Standard ELISA often involve slow reaction rates requiring high incubation times in the range of 1-3hrs. There is a need in the art for a high throughput ELISA which utilises shorter incubation times and can, therefore, be completed in a shorter time period.

### DISCLOSURE OF THE INVENTION

The speed of a reaction with two or more reactants is controlled by two steps: (a) the speed of diffusion of the reactants (characterised by k_{diff}) and (b) the speed of the chemical reaction (characterised by k_{chem}). When both reactants are able to move freely the reaction is called a homogeneous phase reaction (that is, one where at least two reactants are in a fluid phase) and when one reactant is immobilised the reaction is called a heterogeneous phase reaction. As one of the components in a heterogeneous phase reaction has a k_{diff} of zero, the reaction takes place at a slower rate than a homogeneous reaction in which both reactants have a high k_{diff.}

All of the reaction steps in a standard ELISA are heterogeneous phase reactions i.e. one component is in solution whilst the other is immobilised.

The present inventors have surprisingly found that changing one or more of the steps in the ELISA from a heterogeneous phase to a homogeneous phase reaction results in a much shorter overall completion time, allowing an ELISA to be completed in less time than that required for an ELISA known in the art. For example, in some embodiments changing one step to a homogeneous phase reaction allows completion in about 30% of the time required for an ELISA known in the art. In other embodiments, the time may be reduced by about, e.g., 10%, 20%, 40%, 50% or 60%. In addition, changing one step to a homogeneous phase ensures that each binding of the immune complexes to the plate wells is an effective binding in terms of signal development, therefore the incubation time of the heterogeneous phase can be dramatically reduced, or a standard incubation time used to give a high increase in signal amplification. The short ELISA method confers many advantages over the standard ELISA. For example, the increased speed of completion of the short ELISA method allows for the simple, rapid detection of pathogens in a clinical setting and thus allows the provision of results to patients and accordingly treatment of patients over a shorter time period.

In some embodiments of the new short ELISA method for antibodies, serum antibodies (analyte) are mixed with labelled antibodies specific to the serum antibodies in a reaction container, e.g. a test tube, (homogeneous phase) and incubated for 10mins or 5mins (Step 1). The mixing product is then transferred to a microtitre well coated with an antigen and incubated for 10mins or 10-15mins (Step 2). The micro well is washed and a colour reagent added and developed for 30mins or 10mins (Step 3). A final washing step can precede measuring the absorbance at a wave length specific for the colour reagent. Thus the analyte and the label are pre-mixed in a homogeneous phase before meeting the immobilised binding partner for the analyte (i.e. serum antibody in this example). Figure 2 provides a graphical representation of one embodiment of the short ELISA method of the present application for assaying serum antibodies as the analyte. If the analyte were an antigen, then the second reaction container would be coated with antibody as opposed to antigen.

Accordingly the invention provides a binding assay for detecting an analyte in a sample, comprising the steps of (i) mixing in a homogeneous phase the sample and a first binding partner to the analyte to form a first mixing product; and (ii) exposing the first mixing product to a second binding partner to the analyte. Preferably the first binding partner is labelled.

Typically the second binding partner is immobilised such that step (iii) occurs in the heterogeneous phase. For example, step (i) may be carried out in a first reaction container and step (ii) in a second reaction container where the second binding partner is immobilised on the surface of the second reaction container.

Thus the invention provides a binding assay for detecting an analyte in a sample, comprising the steps of (i) mixing in a homogeneous phase, in a first reaction container, the sample and a first binding partner to the analyte to form a first mixing product; (ii) transferring the first mixing product to a second reaction container; and (iii) mixing in the second reaction container the first mixing product and a second binding partner to the analyte. Preferably the first binding partner is labelled.

### Step (i)

Step (i) of the short ELISA method involves mixing, in a homogeneous phase the sample to be tested for an analyte and a first binding partner to the analyte to form a first mixing product. This step takes place in a first reaction container.

A sample may contain or be suspected of containing one or more analytes intended for detection by the short ELISA method. Even if the analyte is absent, however, the assay is still "for detecting" the analyte. For example, the assay may be performed on a negative control sample to confirm the fidelity of the assay. As a further example, if the short ELISA method is used to detect the presence or absence of serum antibodies to a pathogen in a patient, the first mixing product of step (i) may be either an immune complex, in the case of an infected patient, or may be defined by the absence of an immune complex in a non-infected patient.

The choice of binding partner used in step (i) will depend on the analyte. For example where the analyte is a serum antibody then the first binding partner may be a labelled antibody specifically targeted at the serum antibody e.g. at its heavy chain, and the second binding partner may be a polypeptide containing an epitope recognised by the serum antibody. In a second example the analyte may be a polypeptide containing a first epitope recognised by the first binding partner, a labelled antibody, and the second binding partner may be an antibody recognising a second epitope in the polypeptide. In certain embodiments the second binding partner may recognise a hidden or cryptic epitope on the first binding partner. It is envisaged that such an epitope is only presented after a structural change in the first binding partner has occurred. For example a change occurs as a result of the first binding partner binding the analyte. It will be apparent to the person skilled in the art that the binding partner will vary depending on the choice of analyte.

The mixing required in step (i) is in the homogeneous phase and is intended to ensure a uniform composition throughout the solution. This may be achieved by any means including, but not limited to, agitation, mechanical or manual stirring or repeat pipetting.

As in known ELISA methods, the binding reaction can be detected by various means. Typically the first binding partner may be labelled by any means known in the art, in particular those means used in standard ELISA. The first binding partner may be labelled by conjugation of the binding partner to a direct label, for example, a coloured particle, an electrochemically active reagent, a redox reagent, a radioactive isotope, a fluorescent label or a luminescent label. The first binding partner may also be labelled by conjugation of the binding partner to an indirect label, for example, an enzyme.

Where the first binding partner is conjugated to an indirect label this may be an enzyme. The enzyme is preferably capable of causing a detectable change in a reactant e.g. of modifying a reactant in a manner which causes a colour change. For example the enzyme may be a peroxidase, particularly horseradish peroxidase, which can be used with an appropriate substrate to give a coloured reaction product e.g. 3,3',5,5'-tetramethylbenzidine. The indirect label may also be a molecule with a high affinity for another molecule, for example avidin. In this case, exposure of the first binding partner to a labelled biotin molecule would allow its detection.

Where the first binding partner is conjugated to a direct label, the direct label is an entity, which is detectable in its natural state. For example, where the direct label is a coloured particle, such as dye sols, metallic sols (e.g. gold), and coloured latex particles, this may be visible to the naked eye, or become visible with the aid of an optical filter. Where the direct label is a fluorescent label, this may be subjected to applied stimulation, e.g. UV light to promote fluorescence.

The second binding partner is normally not labelled. However, the second binding partner may be labelled, for example, using a method such as FRET detection. In this case the first binding partner will be labelled with either a donor or acceptor dye, whilst the second binding partner will be labelled with the other. Only when the two first and second binding partner are brought into close proximity, through their respective binding of the analyte, will photon emission be observable.

Antibodies used in the assay may be either polyclonal or monoclonal and may be produced by any method known in the art.

Antibodies for use for detection may be of any isotype (e.g. IgA, IgG, IgM i.e. an α, γ or µ heavy chain). Antibodies may have a κ or a λ light chain. Within the IgG isotype, antibodies may be IgG1, IgG2, IgG3 or IgG4 subclass. In embodiments of the short ELISA method where the first binding partner is a conjugated antibody then the antibody is intended to be used at a concentration in the range of about 1:100 to 1:12000, e.g. about 1:100, 1:200, 1:300, 1:500, 1:1000, 1:1500, 1:2000, 1:3000, 1:4000, 1:5000, 1:6000, 1:7000, 1:8000, 1:9000, 1:10000, 1:12000.

Suitable reaction containers for use in the invention can hold a volume of liquid and include, but are not limited to, test tubes, eppendorf tubes, wells in microtitre plates and mixing trays.

Step (i) of the short ELISA method is intended to be incubated for a finite period of time in the range of about 1 to 300 minutes, e.g. about 1, 2, 5, 10, 20, 40, 60, 120, 180, 240 or 300. Most preferably step (i) is incubated for about 10 minutes or about 5 minutes.

Step (i) may be incubated at any temperature in the range of about 0-99°C, e.g. about 1, 2, 3, 5, 10, 15, 20, 25, 30, 35, 37, 40, 50, 60, 80 or 100°C. Most preferably step (i) is incubated at about 37°C.

### Step (ii)

Step (ii) of the short ELISA method involves exposing the first mixing product to a second binding partner to the analyte. This step can take place in the same reaction container or may involve the transfer of the first mixing product to a second reaction container.

If step (ii) of the short ELISA takes place in the same reaction vessel, then the exposure of the first mixing product to the second binding partner may be facilitated by the addition of a slide, rodor beads, coated with the second binding partner, to the first reaction vessel.

If step (ii) of the short ELISA involves the transfer of the first mixing product to a second reaction container then this transfer can be achieved by any method known in the art including, but not limited to, pipetting, pouring and transfer along microchannels between wells in a microfluidic chip.

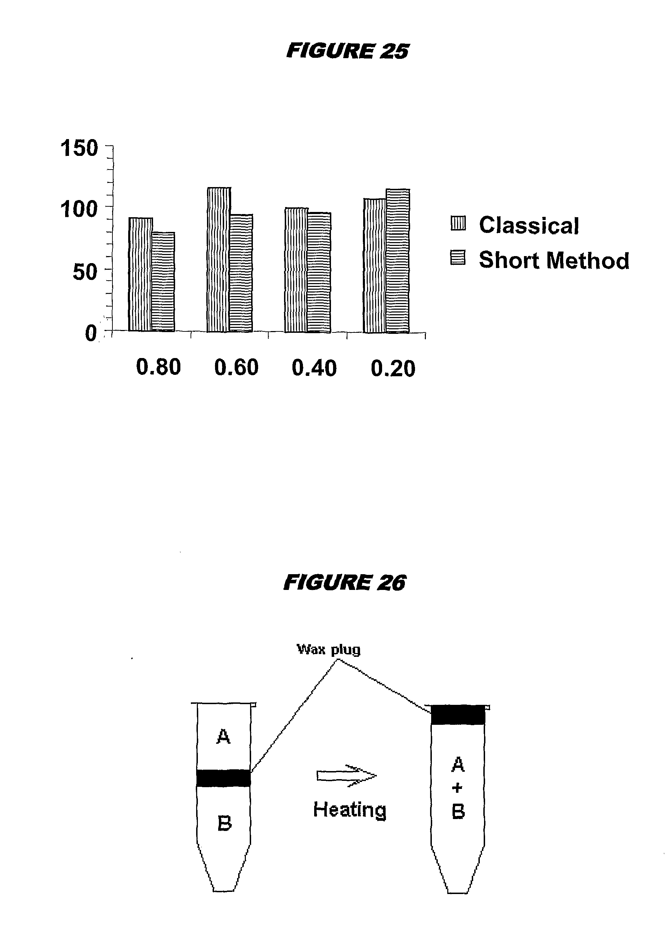

Also envisaged by the invention is a first reaction container, separated from the second reaction container by a removable partition. The partition prevents the mixing product of step (i) gaining access to the second binding partner until step (ii). In such a case, transfer of the first mixing product to the second reaction container involves the removal of the partition. For example, the mixing container may be an eppendorf tube containing a wax plug separating the reagents used in step (i) and step (iii) of the reaction. Transfer of the first mixing product between the first and second reaction container in this case is achieved by increasing the temperature of the tube sufficiently to melt the wax plug (Figure 26).

Using the standard ELISA method all reactions occur in the heterogeneous phase. In the short ELISA method of the present invention, the mixing of the first binding partner and the sample containing the analyte are intended to take place in a homogeneous phase reaction. However, the mixing of the first mixing product with the second binding partner (the second mixing reaction) may occur in either a homogeneous or, preferably, a heterogeneous phase reaction.

For the second mixing reaction to occur in a heterogeneous phase it is envisaged that the second binding partner will be immobilised. In some embodiments, the second binding partner is immobilized on a substrate. The substrate may be any surface or support upon which the second binding partner can be immobilised, including one or more of a solid support (e.g., glass such as a glass slide or a coated plate, silica, plastic or derivatized plastic, paramagnetic or non-magnetic metal), a semi-solid support (e.g., a polymeric material, a gel, agarose, or other matrix), and/or a porous support (e.g., a filter, a nylon or nitrocellulose membrane or other membrane). In some embodiments, synthetic polymers can be used as a substrate, including, e.g., polystyrene, polypropylene, polyglycidylmethacrylate, aminated or carboxylated polystyrenes, polyacrylamides, polyamides, polyvinylchlorides, and the like. In preferred embodiments, the substrate comprises a microtiter immunoassay plate or other surface suitable for use in an ELISA.

The surface of the substrate or support may be planar, curved, spherical, rod-like, pointed, wafer or wafer-like, or any suitable two-dimensional or three-dimensional shape on which the second binding partner may be immobilised, including, e.g., films, beads or microbeads, tubes or microtubes, wells or microtiter plate wells, microfibers, capillaries, a tissue culture dish, magnetic particles, pegs, pins, pin heads, strips, chips prepared by photolithography, etc. In some embodiments, the surface is UV-analyzable, e.g., UV-transparent.

Immobilisation may be achieved in any number of ways, known in the art, described herein, and/or as can be developed. For example, immobilisation may involve any technique resulting in direct and/or indirect association of an analyte (and its corresponding antagonist) with the substrate, including any means that at least temporarily prevents or hinders its release into a surrounding solution or other medium. The means can be by covalent bonding, non-covalent bonding, ionic bonding, electrostatic interactions, Hydrogen bonding, van der Waals forces, hydrophobic bonding, or a combination thereof. For example, immobilisation can be mediated by chemical reaction where the substrate contains an active chemical group that forms a covalent bond with the second binding partner. For example, an aldehyde-modified support surface can react with amino groups in protein receptors; or amino-based support surface can react with oxidization-activated carbohydrate moieties in glycoprotein receptors; a support surface containing hydroxyl groups can react with bifunctional chemical reagents, such as N,N dissuccinimidyl carbonate (DSC), or N-hydroxysuccinimidyl chloroformate, to activate the hydroxyl groups and react with amino-containing receptors. In some embodiments, support surface of the substrate may comprise animated or carboxylated polystyrenes; polyacrlyamides; polyamines; polyvinylchlorides, and the like. In still some embodiments, immobilization may utilize one or more binding-pairs to bind or otherwise attach a receptor to a substrate, including, but not limited to, an antigen-antibody binding pair, hapten/anti-hapten systems, a avidin-biotin binding pair; a streptavidin-biotin binding pair, a folic acid/folate binding pair; photoactivated coupling molecules, and/or double stranded oligonucleotides that selectively bind to proteins, e.g., transcriptional factors.

The second binding partner may be immobilised on the surface of the second reaction container or to beads within the second reaction container or added to the first reaction container (e.g. superparamagnetic polystyrene beads - Dynabeads M-450). Once binding of the first mixing product to second binding partner is achieved, unbound reactants can be washed from the immobilised second mixing product. Detection of the second mixing product is then possible. The second mixing reaction may also occur in the homogeneous phase. For example, binding of the second binding partner to the first mixing product may induce a precipitation of the second mixing product. Such a precipitation would allow the separation of the second mixing product from the unbound reactants. Such separation could be facilitated by any method known in the art including, but not limited to, centrifugation or filtration.

If the reaction of step (ii) is carried out in a homogeneous phase, then mixing is intended to ensure a uniform composition throughout the solution. If the reaction of step (ii) is carried out in a heterogeneous phase with the second binding partner immobilised on the surface of the second reaction container then it is not possible to achieve a uniform composition. In this case, mixing may include agitation of the second reaction container to increase the chance of reactants coming into contact with each other.

Step (ii) of the short ELISA method is intended to be incubated for a finite period of time in the range of about 1 to 300 minutes, e.g. about 1, 2, 5, 10, 20, 40, 60, 120, 180, 240 or 300. Most preferably step (ii) is incubated for about 10 minutes or about 10-15 minutes.

Step (ii) may be incubated at any temperature in the range of about 0-99°C, i.e. about 1, 2, 3, 5, 10, 15, 20, 25, 30, 35, 37, 40, 50, 60, 80 or 100°C. Most preferably step (ii) is incubated at about 37°C.

### Step (iii)

The method may include a further step (iii). Step (iii) of the short ELISA method involves the optional detection of a second mixing product comprising the first binding partner, the analyte and the second binding partner.

In a preferred embodiment, the detection of the second mixing product is achieved through detection of a colour change in a reagent mixed with the second mixing product.

The reaction of step (iii) of the short ELISA method is intended to be incubated for a finite period of time in the range of about 1 to 300 minutes, e.g. about 1, 2, 5, 10, 20, 40, 60, 120, 180, 240 or 300. Most preferably step (iii) is incubated for about 30 minutes or about 10 minutes.

The reaction of step (iii) may be incubated at any temperature in the range of about 0-99°C, e.g. about 1, 2, 3, 5, 10, 15, 20, 25, 30, 35, 37, 40, 50, 60, 80 or 100°C. Most preferably step (iii) is incubated at about 37°C.

Where step (iii) involves detecting a colour change to p-NNP, the p-NPP concentration may be in the range of about 0.1mg/ml to 20mg/ml, e.g. about 0.1, 0.2, 0.5, 1.0, 2.0, 3.0, 4.0, 6.0, 8.0, 10.0, 15.0 or 20mg/ml. Most preferably the p-NNP concentration is about 3.0mg/ml.

The short ELISA method is intended to take a finite period of time in the range of about 1 to 300 minutes, e.g. about 1, 2, 5, 10, 15, 20, 25, 30, 35, 40, 45, 50, 55, 60, 100, 120, 150, 180, 240 or 300. Most preferably the short ELISA method takes less than one hour.

### Samples and analytes

A sample for use in the invention can refer to specimen material used for a given assay, reaction, run, trial, and/or experiment. For example, a sample may comprise an aliquot of the specimen material collected, up to and including all of the specimen. Samples may be crude samples or processed samples, e.g., obtained after various processing or preparation steps carried out on the original specimen. For example, various cell separation methods, e.g., magnetically activated cell sorting, may be applied to separate or enrich analytes of interest in a biological fluid, such as blood. A sample may also comprise a dilution of a specimen, e.g., diluted serum or dilutions of other complex and/or protein-rich mixtures. For example, in some embodiments, a specimen may be serially diluted to provide a number of serially-diluted samples for analysis. As used herein the terms assay, reaction, run, trial and/or experiment can be used interchangeably. Preferred embodiments of the present invention can be practiced using small starting amounts of analytes to yield quantifiable results.

Samples for use in step (i) may be taken from an *in vivo* or *in vitro* source. Examples of *in vivo* sources include blood extracts, serum, cell extracts or homogenised tissue taken from a patient. Examples of *in vitro* sources include tissue culture extracts or growth media extracts from a variety of cell cultures including animal cell lines, hybridomas, yeasts and bacteria.

Analytes for use in the invention include but are not limited to e.g., small molecules such as small natural or synthetic organic molecules of up to 2000Da, preferably 800Da or less; peptidomimetics; inorganic molecules; drugs or pharmaceuticals; cytokines; toxins; macromolecular structures; metabolites; natural or modified substrates; steroids; enzymes; hormones; nucleic acids; proteins; receptors; peptides; glycoproteins; domains or motifs; amino acids; lectins; lipids; carbohydrates; sugars; polymers; tissues; cells; cell surface components; cellular components; subcellular organelles; whole or parts of microbes (such as pathogens, parasites, viruses, bacteria, fungi, and the like) from any known organism, more particularly any known virus, bacteria or fungus, in particular HIV, HCV, HBV, Influenza, Rhinovirus, Picornavirus, Poliovirus, E.coli, Helicobacter sp., Enterobacter sp., Salmonella sp., Chlamydia sp., Enterococcus sp., Staphylococcus sp., Streptococcus sp., Bacillus sp., Neisseria sp. Candida sp., Cryptococcus sp., Aspergillus sp; biological and chemical warfare agents; and/or antibodies e.g. to one or more of the above described molecule, including IgG, IgA, IgM, IgD and IgE; structural or functional mimetics of the aforementioned or any combinations thereof.

An analyte as used in the invention will usually be at a concentration of about 0.1ng/ml to 10mg/ml, e.g. about 0.1ng/ml, 1.0ng/ml, 10 ng/ml, 100 ng/ml, 1.0µg/ml, 10 µg/ml, 100 µg/ml, 1.0mg/ml or 10mg/ml.

### General

The term "comprising" encompasses "including" as well as "consisting" *e.g*. a composition "comprising" X may consist exclusively of X or may include something additional *e.g*. X + Y.

The term "about" in relation to a numerical value *x* means, for example, *x*±10%.

### BRIEF DESCRIPTION OF DRAWINGS

Figure 1 depicts the steps involved in a standard ELISA.
Figure 2 depicts the steps involved in an embodiment of the short ELISA method.
Figure 3 shows a plot of recovery (%) vs. serum reciprocal dilution.
Figure 4 shows a plot of A₄₀₅nm vs. serum reciprocal dilution.
Figures 5 shows a plot of recovery vs. nominal recovery (%) at different conjugated antibody reciprocal concentrations.
Figure 6 shows a comparison of recovery (%) vs. preincubation time of the homogeneous phase reaction.
Figure 7 shows a plot of A₄₀₅nm vs. AU/ml with a logistic regression for an immune complex incubation time of 1hr at 37°C.
Figure 8 shows a plot of A₄₀₅nm vs. AU/ml with a line of best fit.
Figure 9 shows a plot of A₄₀₅nm vs. AU/ml with a linear regression for an immune complex incubation time of 10min at 37°C.
Figure 10 shows plots of A₄₀₅nm vs. AU/ml for different immune complex incubation times
Figure 11 shows a bar chart representation of the sum of deviations from a linear calibration function vs. immune complex incubation times
Figure 12 shows a bar chart representation of the sum of deviations from a linear calibration function vs. p-NPP concentration
Figures 13-16 show plots of A₄₀₅nm vs. AU/ml for different p-NPP concentrations where all other experimental variables have been kept constant.
Figures 17-19 show plots of A₄₀₅nm vs. AU/ml for different immune complex incubation times using a p-NPP concentration of 3.0mg/ml
Figure 20 shows a bar chart representation of the sum of deviations from a linear calibration function vs. immune complex incubation time where the concentration of p-NPP is 3.0mg/ml Figure 21 shows a plot of A₄₀₅nm vs. AU/ml using a standard for accuracy verification.
Figures 22 shows a plot of A₄₀₅nm vs. AU/ml for a comparison between the standard and short ELISA using OMV
Figure 23 shows a bar chart representation of the recovery (%) vs. expected values for a comparison between the standard and short ELISA using OMV
Figure 24 shows a bar chart representation of the recovery (%) vs. expected values for a comparison between the standard and short ELISA using 287
Figure 25 shows a bar chart representation of the recovery (%) vs. expected values for a comparison between the standard and short ELISA using GBS
Figure 26 shows a preferred embodiment of a first reaction container, separated from the second reaction container by a removable partition, in this case a wax plug.

### MODES FOR CARRYING OUT THE INVENTION

### Example 1 - Optimisation of the short ELISA procedure

A number of aspects of an embodiment of a rapid ELISA assay for various different antigens were optimised experimentally with regard to assaying serum antibodies using a conjugated antibody to the serum antibodies as the first binding partner, p-nitrophenylphosphate (p-NPP) as the detection means and a protein as the second binding partner.

### Serum dilution optimization

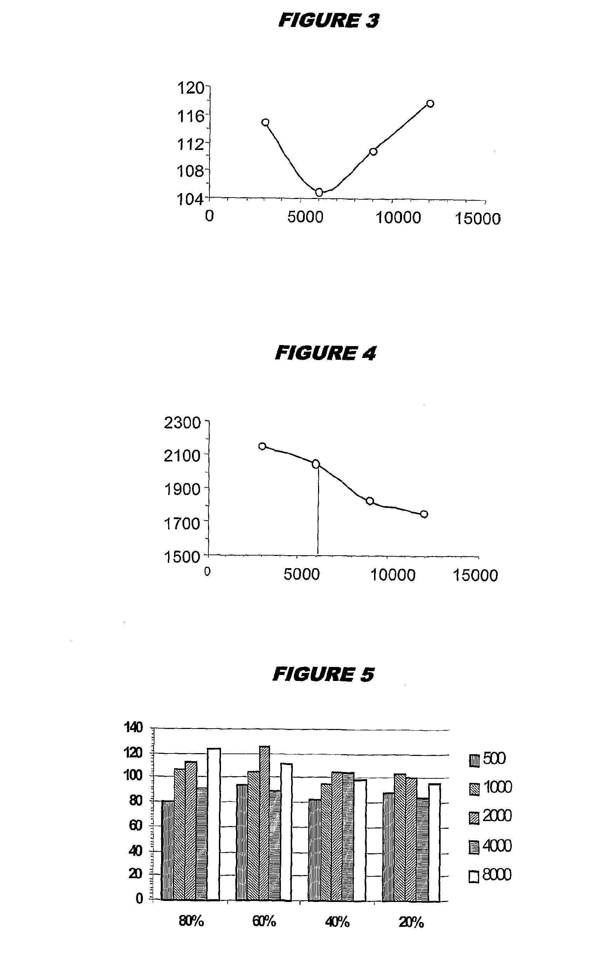

To optimise the serum dilution concentration used in the short ELISA a number of experiments were conducted and the mean recovery (%) and sensitivity were compared over a range of serum dilutions from 1:3000-1:12000. The results are shown in Table 1 and Figures 3 and 4:

**Table 1**

| Serum Antibody dilution | Mean Recovery (%) | Sensitivity |
|---|---|---|
| 3000 | 115 | 2150 |
| 6000 | 105 | 2049 |
| 9000 | 111 | 1826 |
| 12000 | 118 | 1751 |

In this experiment, a serum dilution of 1:6000 was chosen as this dilution gives the best recovery whilst maintaining a good sensitivity.

### Optimization of the dilution of the conjugated antibody

To optimise the dilution of the conjugated antibody used in the short ELISA the recovery (%) vs nominal recovery (%) was compared over a range of conjugated antibody dilutions. The serum antibody dilution was kept constant at 1:6000. The results are shown in Table 2 and Figure S.

**Table 2**

| Conjugated Antibody dilution | 80% | 60% | 40% | 20% | Mean Recovery (%) |
|---|---|---|---|---|---|
| 500 | 80 | 94 | 82 | 88 | 82 |
| | | | | | |
| 1000 | 107 | 105 | 95 | 104 | 103 |
| 2000 | 113 | 125 | 105 | 100 | 111 |
| 4000 | 91 | 89 | 105 | 85 | 93 |
| 8000 | 123 | 112 | 99 | 96 | 108 |

In this experiment, a 1:1000 dilution of the conjugated antibody gave the best agreement between recovery and nominal recovery.

### Optimization of the incubation time to form the first mixing product of the homogeneous phase reaction

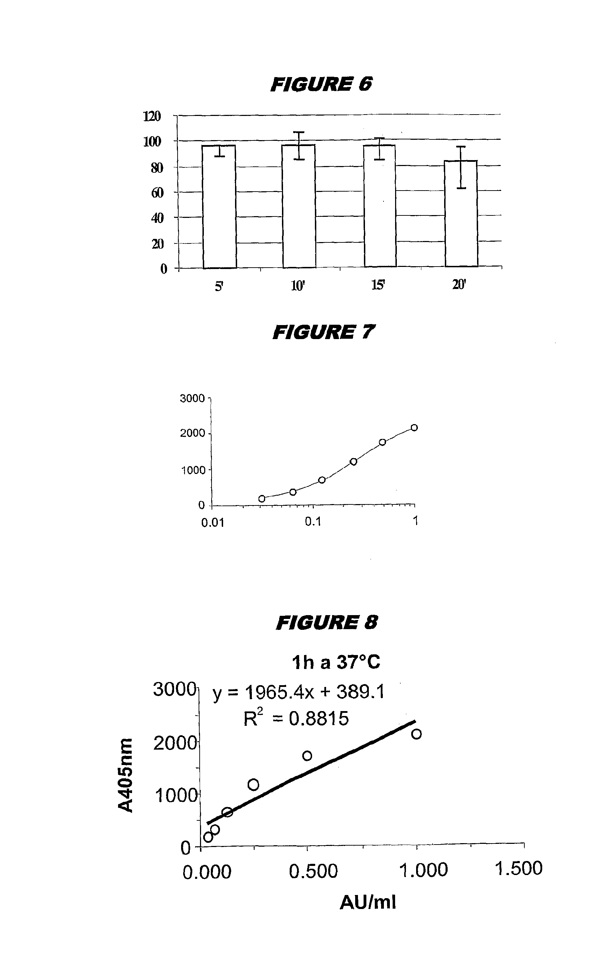

To optimise the incubation time of the homogeneous phase reaction used in the short ELISA average recovery (%) were tested over a range of times. The results are shown in Table 3 and Figure 6.

**Table 3**

| Incubation time (mins) | Average recovery (%) |
|---|---|
| 5 | 96 |
| 10 | 96 |
| 15 | 96 |
| 20 | 83 |

Times ranging from 5mins to 15mins were deemed to be a suitable choice for the homogeneous phase incubation. In this experiment, a 10min or a 5min incubation period was chosen as a good compromise of accuracy and test speed.

### Optimization of the incubation time to form the second mixing product (the immune complex)

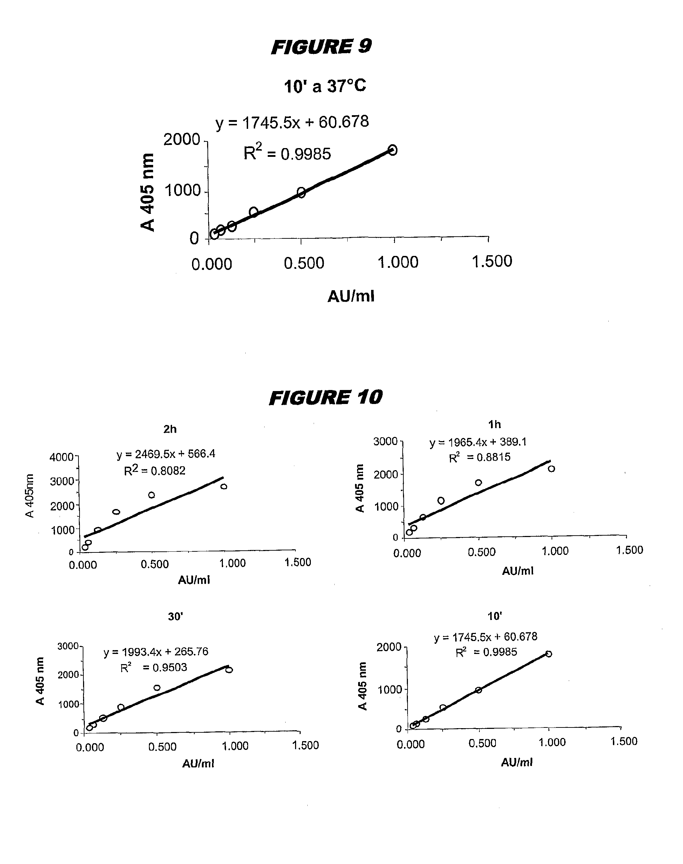

The incubation time for the immune complex of 1hr at 37°C gave good results. However, the data using this incubation time required a non-linear, logistic regression, calibration function. In order to achieve a linear calibration different incubation times were tried (Figures 7-11, Table 4). It was found that linearity of the calibration function could be achieved using an incubation time of 10mins at 37°C.

The reason for the non-linearity of the calibration function at longer incubation times was investigated. It was proposed that at shorter incubation times less immune complex bound to the plate and so the colour developing reagent (pNPP) remains in excess. However, with longer incubation times the substrate is exhausted and so the linear regression is not maintained. To confirm this hypothesis the effect of p-NPP concentration was investigated.

**Table 4**

| Incubation time of immune complex (mins. at 37°C) | Sum of deviations from linear expected values |
|---|---|
| 10 | 167 |
| 15 | 410 |
| 30 | 1784 |
| 60 | 2469 |
| 120 | 4525 |
| 180 | 4739 |

### Optimization of the p-nitrophenylphosphate (p-NP) concentration

To optimise the p-NPP concentration used in the colour developing solution the concentration was varied from 1.0mg/ml through 4.0mg/ml. The experiments were carried out using a 1:6000 serum dilution, a 1:1000 dilution of ALP conjugated anti-mouse antibody, a 10min incubation period for the homogeneous phase followed by a 30min incubation period of the immune complex (shown to be non-linear with 1.0mg/ml p-NPP in previous experiments).

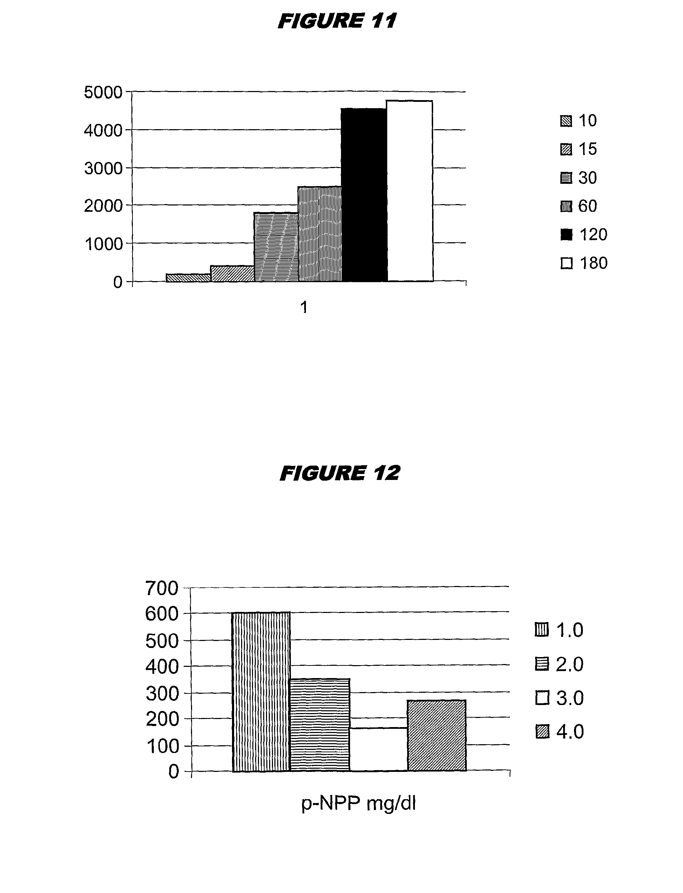

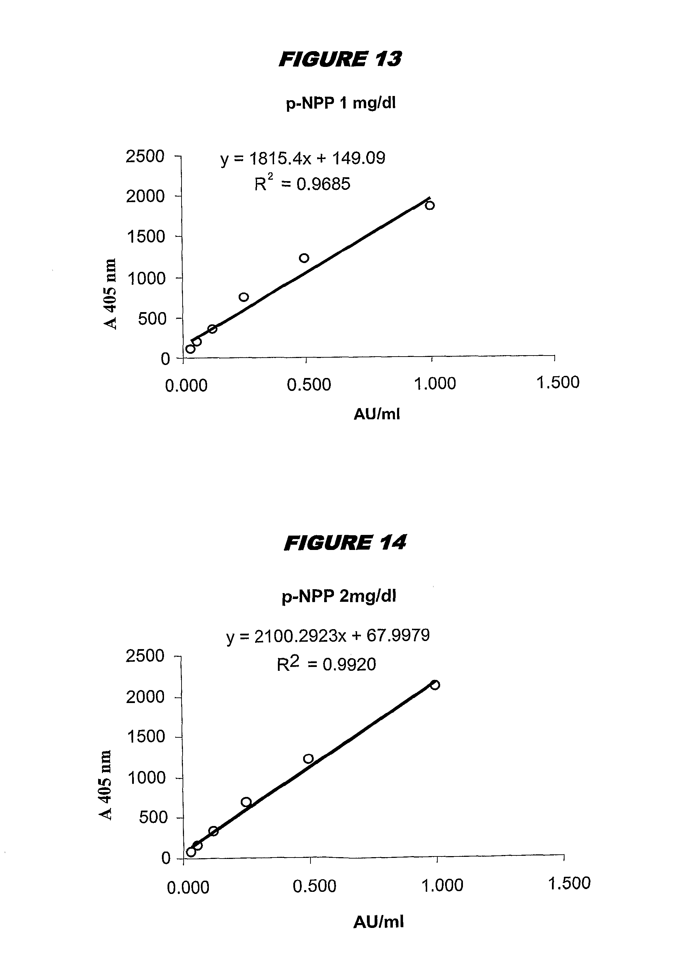

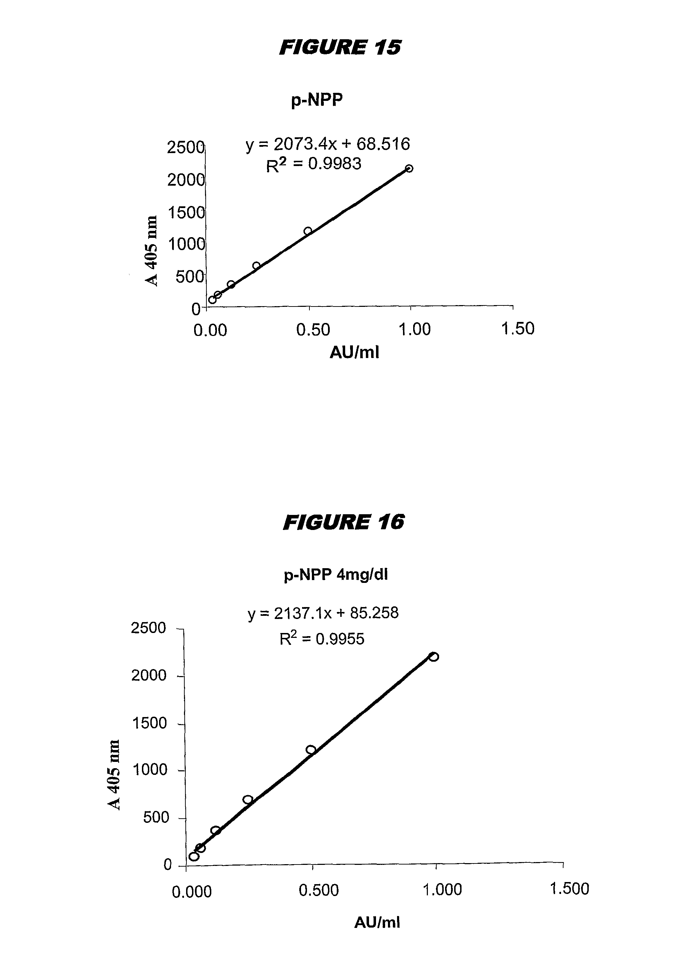

The sum of the deviations from a linear calibration function were measured over the range of p-NPP concentrations (Table 5, Figures 12-16). In this experiment, the optimum p-NPP concentration, with a 30min incubation period for the immune complex, was shown to be 3.0mg/ml. Having deduced the optimum p-NPP concentration a reduction in immune complex incubation time with this concentration was investigated.

**Table 5**

| p-NNP mg/ml | Sum of deviations from linearity |
|---|---|
| 1.0 | 603 |
| 2.0 | 347 |
| 3.0 | 163 |
| 4.0 | 263 |

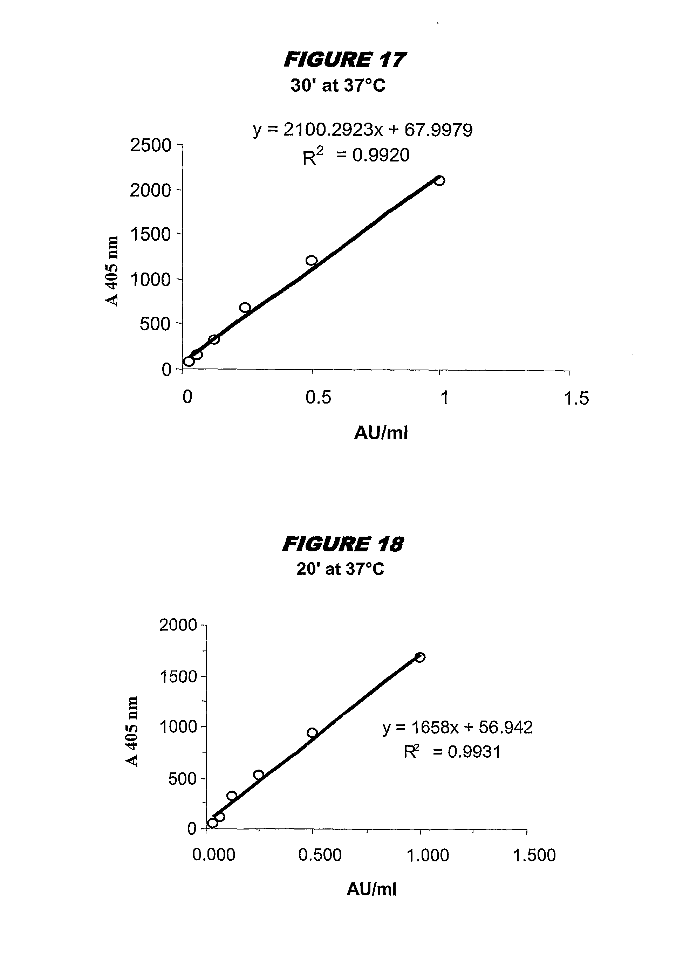

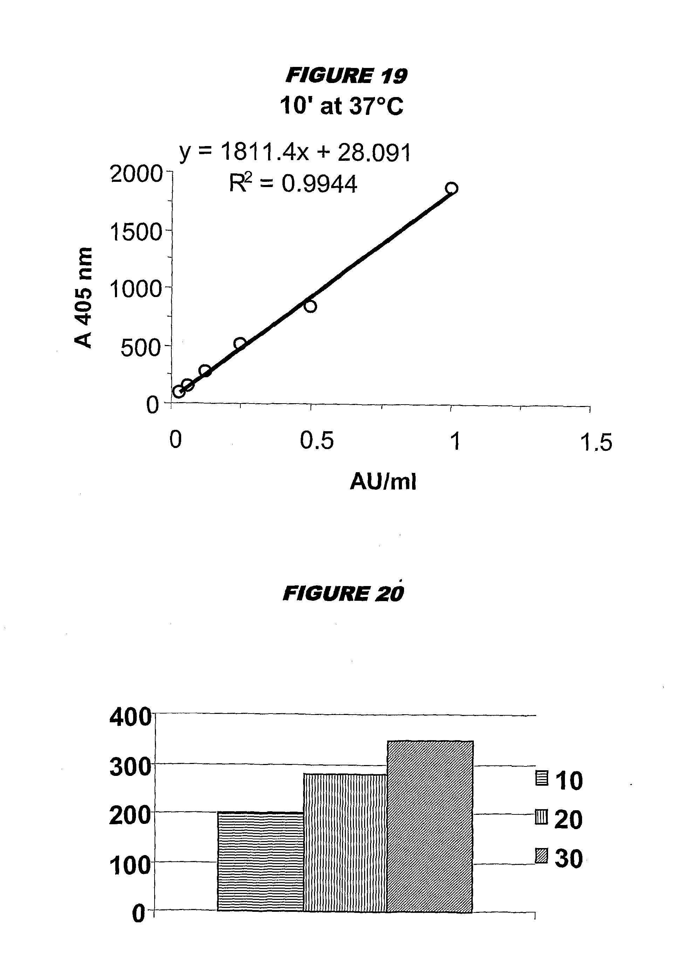

Using a p-NPP concentration of 3.0mg/ml, three immune complex incubation times were tested (Tables 10-12, Figure 17-20). In this experiment, the optimum conditions were shown to be an immune complex incubation time of 10mins or 10-15mins with a p-NPP concentration of 3.0mg/ml followed by 30mins or 10mins of colour development.

**Table 10 - 30min at 37°C**

| Mean | Deviations |
|---|---|
| 2109 | 59 |
| 1205 | 87 |
| 680 | 87 |
| 323 | 8 |
| 152 | 45 |
| 71 | 61 |
| Sum | **347** |

**Table 11- 20min at 37°C**

| Mean | Deviations |
|---|---|
| 1679 | 36 |
| 932 | 46 |
| 518 | 46 |
| 311 | 47 |
| 115 | 46 |
| 51 | 58 |
| Sum | **279** |

**Table 12 - 10min at 37°C**

| Mean | Deviations |
|---|---|
| 1881 | 41 |
| 834 | 100 |
| 503 | 22 |
| 274 | 20 |
| 150 | 9 |
| 93 | 8 |
| Sum | **200** |

### Example 2 - Verification of the short ELISA test through accuracy trial

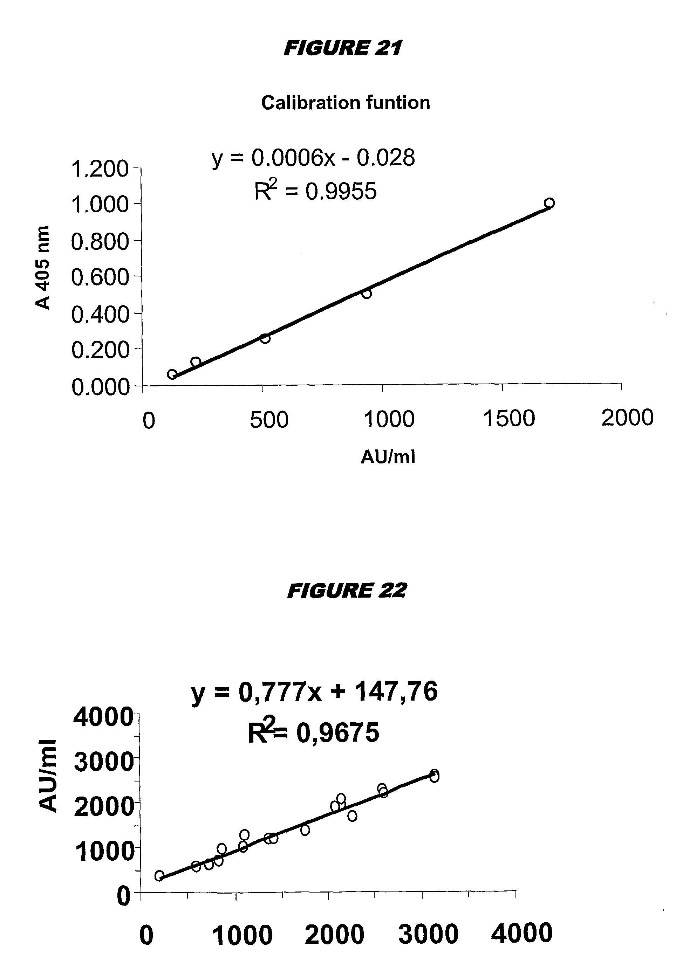

Once optimum conditions had been established for the short ELISA procedure in these experiments, the test was verified in an accuracy trial. It was found that linear calibration was maintained over a range of standard concentrations (Table 13-15, Figure 21).

**Table 13**

| Plate for accuracy verification | | | | | | | | | |
|---|---|---|---|---|---|---|---|---|---|
| Std | | 80% of std | | 60% of std | | 40% of std | | 20% of std | |
| 1676 | 1738 | 1482 | 1616 | 1149 | 1159 | 827 | 788 | 453 | 486 |
| 949 | 938 | 770 | 824 | 627 | 581 | 371 | 379 | 236 | 224 |
| 520 | 513 | 414 | 405 | 332 | 316 | 216 | 220 | 113 | 111 |
| 216 | 237 | 190 | 246 | 152 | 138 | 102 | 99 | 45 | 50 |
| 97 | 154 | 144 | 137 | 77 | 70 | 66 | 47 | 31 | 15 |
| 46 | 37 | 64 | 113 | 17 | 83 | 75 | 58 | 39 | 20 |
| 16 | 40 | 11 | 26 | 42 | 44 | 45 | 40 | 19 | 70 |
| 57 | 49 | 27 | 50 | 76 | 83 | 80 | 71 | 55 | 22 |

**Table 14**

| Std | AU/ml |
|---|---|
| 1852 | 1.000 |
| 1038 | 0.500 |
| 630 | 0.250 |
| 265 | 0.125 |
| 139 | 0.063 |

**Table 15**

| % | A 405nm | AU/ml | Recovery % |
|---|---|---|---|
| 80 | 1670 | 0.87 | |
| | 900 | 0.90 | |
| | 475 | 0.87 | |
| | 240 | 0.71 | |
| | | **0.84** | 104.9 |
| 60 | 1193 | 0.61 | |
| | 674 | 0.65 | |
| | 355 | 0.61 | |
| | | **0.62** | 104.0 |
| 40 | 858 | 0.43 | |
| | 377 | 0.33 | |
| | 210 | 0.29 | |
| | | **0.38** | 94.4 |
| 20 | 532 | 0.25 | |
| | 228 | 0,16 | |
| | | **0.21** | 103.2 |
| Mean recovery % | | | 101.6 |

### Example 3 - Verification of the short ELISA test through comparison trial

Once the accuracy of the short ELISA method had been confirmed, further verification was carried out in a series of comparisons with standard ELISA. Initially the two methods were compared using outer membrane vesicles (OMVs) prepared from serogroup B meningococcus. (Table 16, Figure 22)

**Table 16**

| Standard method AU/ml | Short ELISA method AU/ml |
|---|---|
| 3154 | 2575 |
| 3154 | 2529 |
| 2602 | 2271 |
| 2612 | 2192 |
| 2154 | 1927 |
| 2160 | 2022 |
| 2084 | 1862 |
| 2271 | 1668 |
| 1759 | 1360 |
| 1373 | 1160 |
| 1421 | 1153 |
| 1105 | 1259 |
| 1095 | 995 |
| 874 | 940 |
| 840 | 698 |
| 728 | 595 |
| 596 | 578 |
| 197 | 327 |

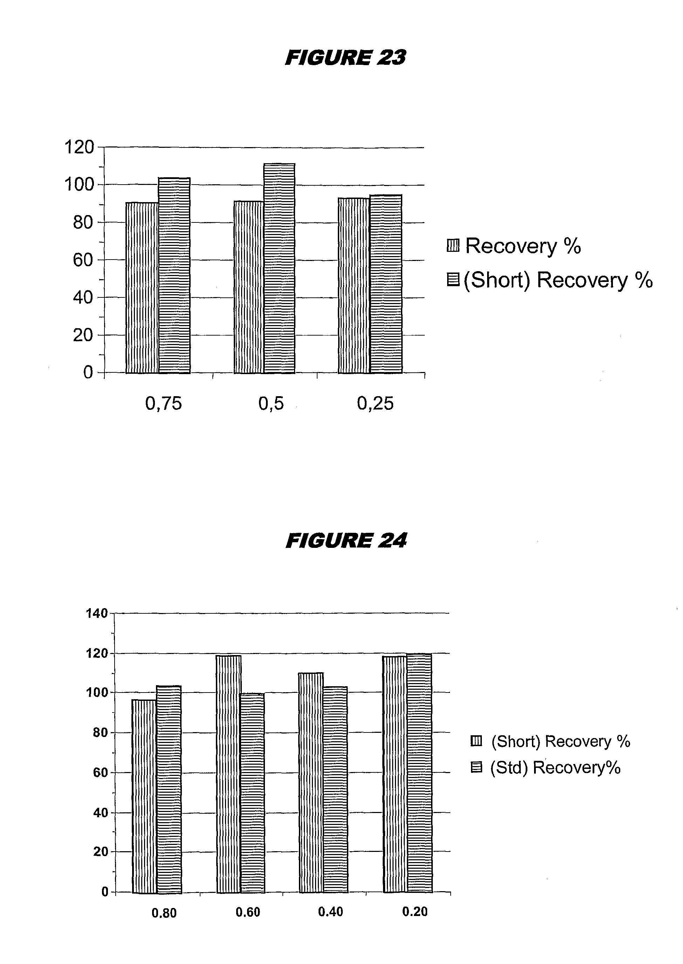

Further comparison testing was carried out using OMVs, protein `287' from meningococcus and 'GBS protein 80' from streptococcus (Tables 17-19, Figures 23-25).

**Table 17 - OMV ELISA**

| Standard Method | | Short Method |
|---|---|---|
| Expected Values | Recovery % | Recovery % |
| 0.75 | 90.7 | 103.3 |
| 0.50 | 91.4 | 111.1 |
| 0.25 | 93.2 | 94.8 |

**Table 18 - 287 ELISA**

| Standard Method | | Short Method |
|---|---|---|
| Expected Values | Recovery % | Recovery % |
| 0.80 | 104 | 96 |
| 0.60 | 100 | 119 |
| 0.40 | 103 | 110 |
| 0.20 | 119 | 118 |

**Table 19 -GBS Protein 80 ELISA**

| Standard Method | | Short Method |
|---|---|---|
| Expected Values | Recovery % | Recovery % |
| 0.80 | 91 | 80 |
| 0.60 | 116 | 94 |
| 0.40 | 100 | 97 |
| 0.20 | 108 | 116 |

It will be understood that the invention has been described by way of example only and modifications may be made whilst remaining within the scope of the invention.

The invention also provides the following numbered embodiments.
1. A binding assay for an analyte in a sample, comprising the steps of:
   (i) mixing in a homogeneous phase, in a first reaction container, the sample and a first binding partner to the analyte to form a first mixing product; and
   (ii) exposing the first mixing product to a second binding partner to the analyte to form a second mixing product
2. The binding assay of embodiment 1, wherein the first binding partner is labelled.
3. The binding assay of embodiment 2, wherein the first binding partner is conjugated to an enzyme.
4. The binding assay of embodiment 2, wherein the first binding partner is conjugated to a direct label.
5. The binding assay of any preceding embodiment, wherein the first binding partner is an antibody.
6. The binding assay of embodiment 5, wherein the antibody is an IgG antibody.
7. The binding assay of embodiment 5 or embodiment 6, wherein the antibody is used at a dilution of 1:1000.
8. The binding assay of any preceding embodiment, wherein the analyte is an antibody.
9. The binding assay of embodiment 8, wherein the antibody is an IgG antibody.
10. The binding assay of embodiment 8 or embodiment 9, wherein the antibody is a serum antibody.
11. The binding assay of embodiment 10, wherein the serum antibody is used at a 1:6000 dilution.
12. The binding assay of any preceding embodiment, wherein step (i) is carried out for 10 minutes or 5 minutes.
13. The binding assay of any preceding embodiment, wherein the second binding partner is immobilised and step (ii) occurs in the heterogeneous phase.
14. The binding assay of any preceding embodiment, wherein step (ii) is carried out for 10 minutes or 10-15 minutes.
15. The binding assay of any one of the preceding embodiments, further comprising detecting said second mixing product.
16. The binding assay of embodiment 15, wherein the detection involves a colour change in a reactant.
17. The binding assay of embodiment 16, wherein the reactant is p-nitrophenylphosphate.
18. The binding assay of embodiment 17, wherein the p-nitrophenylphosphate is at a concentration of 3.0mg/ml.
19. The binding assay of any preceding embodiment, wherein the assay is performed in a total time less than 4hrs.

## Claims

1. A binding assay for an analyte in a sample, comprising the steps of:
(i) mixing in a homogeneous phase, in a first reaction container, the sample and a first binding partner to the analyte to form a first mixing product; and
(ii) exposing the first mixing product to an immobilised second binding partner to the analyte to form a second mixing product.

2. The binding assay of claim 1, wherein the first binding partner is labelled.

3. The binding assay of claim 2, wherein the first binding partner is conjugated to an enzyme.

4. The binding assay of claim 2, wherein the first binding partner is conjugated to a direct label.

5. The binding assay of any preceding claim, wherein the first binding partner is an antibody.

6. The binding assay of claim 5, wherein the antibody is an IgG antibody.

7. The binding assay of claim 5 or claim 6, wherein the antibody is used at a dilution of 1:1000.

8. The binding assay of any preceding claim, wherein the analyte is an antibody.

9. The binding assay of claim 8, wherein the antibody is an IgG antibody.

10. The binding assay of claim 8 or claim 9, wherein the antibody is a serum antibody.

11. The binding assay of claim 10, wherein the serum antibody is used at a 1:6000 dilution.

12. The binding assay of any preceding claim, wherein step (i) is carried out for 10 minutes or 5 minutes.

13. The binding assay of any preceding claim, wherein step (ii) is carried out for 10 minutes or 10-15 minutes.

14. The binding assay of any one of the preceding claims, further comprising detecting said second mixing product.

15. The binding assay of claim 14, wherein the detection involves a colour change in a reactant.

16. The binding assay of claim 15, wherein the reactant is p-nitrophenylphosphate.

17. The binding assay of claim 17, wherein the p-nitrophenylphosphate is at a concentration of 3.0mg/ml.

18. The binding assay of any preceding claim, wherein the assay is performed in a total time less than 4hrs.
